Europäisches Patentamt

European Patent Office

Office européen des brevets

(19)

(11) EP 0 675 717 B1

## (12) EUROPÄISCHE PATENTSCHRIFT

(45) Veröffentlichungstag und Bekanntmachung des Hinweises auf die Patenterteilung:
**11.08.1999 Patentblatt 1999/32**

(21) Anmeldenummer: **94902661.1**

(22) Anmeldetag: **02.12.1993**

(51) Int. Cl.$^6$: **A61K 31/425**

(86) Internationale Anmeldenummer:
**PCT/EP93/03389**

(87) Internationale Veröffentlichungsnummer:
**WO 94/13287 (23.06.1994 Gazette 1994/14)**

(54) **VERWENDUNG VON 2-AMINO-6-A-PROPYL-AMINO-4,5,6,7-TETRAHYDROBENZOTHIAZOL (PRAMIPEXOL) ALS ARZNEIMITTEL MIT ANTIDEPRESSIVER WIRKUNG**

USE OF 2-AMINO-6-A-PROPYLAMINO-4,5,6,7-TETRAHYDROBENZOTHIAZOLE (PRAMIPEXOL) AS AN ANTI-DEPRESSANT DRUG

UTILISATION DE 2-AMINO-6-A-PROPYL-AMINO-4,5,6,7-TETRAHYDROBENZOTHIAZOLE (PRAMIPEXOL) COMME MEDICAMENT A EFFET ANTIDEPRESSEUR

(84) Benannte Vertragsstaaten:
AT BE CH DE DK ES FR GB GR IE IT LI LU MC NL PT SE

(30) Priorität: **05.12.1992 DE 4241013**

(43) Veröffentlichungstag der Anmeldung:
**11.10.1995 Patentblatt 1995/41**

(73) Patentinhaber:
- **BOEHRINGER INGELHEIM INTERNATIONAL GmbH**
  **55218 Ingelheim am Rhein (DE)**
  Benannte Vertragsstaaten:
  **GB IE**
- **Boehringer Ingelheim Pharma KG**
  **55216 Ingelheim am Rhein (DE)**
  Benannte Vertragsstaaten:
  **BE CH DE DK ES FR GR IT LI LU MC NL PT SE AT**

(72) Erfinder:
- **HAMMER, Rudolf**
  **D-55218 Ingelheim (DE)**
- **MIERAU, Joachim**
  **D-55127 Mainz (DE)**
- **LEHR, Erich**
  **D-55425 Waldalgesheim (DE)**

- **BORSINI, Franco**
  **I-50047 Prato (IT)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 186 087         EP-A- 0 417 637**
**DE-A- 3 843 227**

- **PHARMACOPSYCHIATRY (GERMANY), 1992, VOL. 25, NO. 6, PAGE(S) 254-260, Benkert O. et al 'Dopamine autoreceptor agonists in the treatment of schizophrenia and major depression'**
- **EUR. J. PHARMACOL. Bd. 200, Nr. 1 , 1991 Seiten 65 - 72 A.J. CARTER ET AL. 'Pramipexole, a dopamine D2 autoreceptor agonist, decreases the extracellular concentration of dopamine in vivo'**
- **W. PSCHYREMBEL 'KLINISCHES WöRTERBUCH' 1982 , WALTER DE GRUYTER , BERLIN siehe Seite 958 siehe: "Prämenstruelles Syndrom"**

**Beschreibung**

Die vorliegende Erfindung betrifft die Verwendung von 2-Amino-6-n-propyl-amino-4,5,6,7-tetrahydrobenzothiazol, insbesondere seines (-)-Enantiomeren, sowie deren pharmakologisch verträglichen Säureadditionssalze zur Herstellung eines Arzneimittels mit antidepressiver Wirkung.

Die genannten Verbindungen sind als Arzneimittel bekannt, wobei die Behandlung der Schizophrenie und die Behandlung der parkinsonschen Krankheit im Vordergrund stehen. Einzelheiten zur Herstellung dieser Verbindungen - deren (-)-Enantiomeren in der Literatur auch als SND 919 oder Pramipexol bezeichnet wird, können der Europäischen Patentanmeldung EP-A-0 186 087 entnommen werden. Überraschenderweise wurde jetzt gefunden, daß Pramipexol antidepressive Eigenschaften aufweist. Von besonderer Bedeutung in diesem Zusammenhang ist, daß die Verbindung eine unerwartet geringe $\alpha$-Rezeptor-Affinität besitzt und somit bei ausgezeichneter Verträglichkeit eine hohe selektive Wirkung hat. Die bislang nicht bekannte lange Wirkungsdauer (Halbwertszeit 12,5 Stunden) und eine unerwartet hohe Bioverfügbarkeit von über 80 % von Pramipexol erweisen sich als besonderer Vorteil bei der Verwendung als Antidepressivum.

Die antidepressive Wirkung von Pramipexol wurde in prae klinischen Untersuchungen im "forced swimming test" nachgewiesen. Einzelheiten dieses Verhaltenstests sind beispielsweise bei Willner, Psychopharmacology 83, 1 - 16 (1984) und Borsini und Meli, Psychopharmacology 94, 147 - 160 (1988) beschrieben.

Pramipexol (SND 919) wurde gegenüber Amineptine - einem bekannten Antidepressivum - an acht Ratten untersucht. Beide Substanzen wurden in physiologischer Kochsalzlösung gelöst und parenteral verabreicht. Die Ergebnisse sind in der Tabelle 1 dargestellt.

Tabelle 1

| Untersuchungen von SND 919 und Amineptine im "forced swimming test" | | |
|---|---|---|
| Verbindung | Dosis mg/kg | Immobilitätszeit (Sek.) |
| Salzlösung | - | 208 ± 10 |
| SND 919 | 0.03 | 210 ± 14 |
| SND 919 | 0.10 | 165 ± 6 |
| SND 919 | 0.30 | 102 ± 17 |
| Amineptine | 20.00 | 134 ± 19 |

Die vorliegenden Ergebnisse belegen eine deutliche Überlegenheit von SND 919 gegenüber Amineptine.

Pramipexol kann zur Behandlung von Depressionen in Form üblicher galenischer Zubereitungen angewendet werden, die im wesentlichen aus einem inerten pharmazeutischen Träger und einer effektiven Dosis des Wirkstoffes bestehen, wie z.B. Tabletten, Dragees, Kapseln, Oblaten, Pulver, Lösungen, Suspensionen, Emulsionen, Sirupe, Suppositorien usw.. Eine therapeutisch wirksame Einzeldosis der erfindungsgemäß gefundenen Indikation liegt im Bereich zwischen 0,1 und 30 mg, bevorzugt zwischen 1 und 5 mg. Neben einer oralen oder intravenösen Applikation kann Pramipexol auch transdermal angewendet werden. Geeignete Freigabesysteme hierzu sind aus dem Stand der Technik bekannt, beispielsweise aus der Europäischen Patentanmeldung 428 038.

Beispiel 1

Dragéekern mit 5 mg 2-Amino-6-n-propyl-amino-4,5,6,7-tetrahydro-benzthiazol -dihydrochlorid

Zusammensetzung:

1 Dragéekern enthält:

| SND 919 | 5,0 mg |
|---|---|
| Milchzucker | 33,5 mg |
| Maisstärke | 10,0 mg |
| Gelatine | 1,0 mg |
| Magnesiumstearat | 0,5 mg |
| | 50,0 mg |

Herstellungsverfahren

Die Mischung der Wirksubstanz mit Milchzucker und Maisstärke wird mit einer 10 %igen wässrigen Gelatinelösung durch ein Sieb von 1 mm Maschenweite granuliert, bei 40 C getrocknet und nochmals durch obiges Sieb gerieben. Das so erhaltene Granulat wird mit Magnesiumstearat gemischt und zu Dragéekernen verpreßt. Die Herstellung muß in abgedunkelten Räumen vorgenommen werden.

| Kerngewicht: | 50 mg |
|---|---|
| Stempel: | 5 mm, gewölbt |

Die so erhaltenen Dragéekerne werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 100 mg

Beispiel 2

Tropfen mit 5 mg 2-Amino-6-n-propyl-amino-4,5,6,7-tetrahydro-benzthiazol-dihydrochlorid

Zusammensetzung:

100 mlg Tropfsubstanz enthalten:

| p-Hydroxybenzoesäure-methylester | 0,035 g |
|---|---|
| p-Hydroxybenzoesäure-n-propylester | 0,015 g |
| Anisöl | 0,05 g |
| Menthol | 0,06 g |

(fortgesetzt)

| Ethanol rein | 10,0 g |
|---|---|
| SND 919 | 0,5 g |
| Zitronensäure | 0,7 g |
| Natriumphosphat sek. x 2H$_2$O | 0,3 g |
| Natriumcyclamat | 1,0 g |
| Glycerin | 15,0 g |
| Dest. Wasser | ad100,0 ml |

Herstellungsverfahren:

Die p-Hydroxybenzoesäureester, Anisöl sowie Menthol werden in Ethanol gelöst (Lösung I). Die Puffersubstanzen, die Wirksubstanz und Nacriumcyclamat werden in dest. Wasser gelöst Glycerin zugefügt (Lösung II). Lösung I wird in Lösung II eingerührt und die Mischung mit dest. Wasser auf das gegebene Volumen aufgefüllt. Die fertige Tropflösung wird durch ein geeignetes Filter filtriert. Die Herstellung und Abfüllung der Tropflösung muß unter Lichtschutz und unter Schutzbegasung erfolgen.

Beispiel 3

Suppositotiren mit 10 mg 2-Amino-6-n-propyl-amino-4,5,6,7-tetrahydro-benzthiazol-dihydrochlorid

1 Zäpfchen enthält:

| SND 919 | 10,0 mg |
|---|---|
| Zäpfchenmassen (z.B. Witepsol W 45) | 1690,0 mg |
|  | 1700,0 mg |

Herstellungsverfahren:

Die feingepulverte Substanz wird mit Hilfe eines Eintauchhomogenisators in die geschmolzene und auf 40°C abgekühlte Zäpfchenmassen eingerührt. Die Masse sind bei 35°C in leicht vorgekühlte Formen ausgegossen.

Zäpfchengewicht:   1,7 g

Beispiel 4

Ampullen mit 5 mg 2-Amino-6-n-propyl-amino-4,5,6,7-tetrahydro-benzthiazol-dihydrochlorid

1 Ampulle enthält:

| SND 919 | 5,0 mg |
|---|---|
| Zitronensäure | 7,0 mg |

(fortgesetzt)

| | |
|---|---|
| Natriumphosphat sek. x $2H_2O$ | 3,0 mg |
| Natriumpyrosulfit | 1,0 mg |
| Dest. Wasser          ad | 1,0 ml |

Herstellungsverfahren:

In ausgekochtem und unter $CO_2$-Begasung abgekühltem Wasser werden nacheinander die Puffersubstanzen, die Wirksubstanz sowie Natriumpyrosulfit gelöst. Man füllt mit abgekochtem Wasser auf das gegebene Volumen auf und filtriert pyrogenfrei.

Abfüllung:          in braune Ampullen Unter Schutzbegasung

Sterilisation:       20 Minuten bei 120°C

Die Herstellung und Abfüllung der Ampullenlösung muß in abgedunkelten Räumen vorgenommen werden.

Beispiel 5

Dragées mit 10 mg 2-Amino-6-n-propyl-amino-4,5,6,7-tetrahydro-benzthiazol-dihydrochlorid

1 Dragéekern enthält:

| | |
|---|---|
| SND 919 | 10,0 mg |
| Milchzucker | 35,5 mg |
| Maisstärke | 12,0 mg |
| Gelatine | 1,0 mg |
| Magenesiumstearat | 0,5 mg |
| | 59 mg |

Herstellungsverfahren:

Analog Beispiel 1.

**Patentansprüche**

1.   Verwendung von 2-Amino-6-n-propyl-amino-4,5,6,7-tetrahydrobenzothiazol sowie seiner pharmakologisch verträglichen Säureadditionssalze zur Herstellung eines Arzneimittels zur Behandlung von Depressionen.

2.   Verwendung von (-)-2-Amino-6-n-propyl-amino-4,5,6,7-tetrahydrobenzothiazol sowie seiner pharmakologisch verträglichen Säureadditionssalze zur Herstellung eines Arzneimittels zur Behandlung von Depressionen.

**Claims**

1.   The use of 2-amino-6-n-propyl-amino-4,5,6,7-tetrahydrobenzothiazol and its pharmacologically-acceptable acid addition salts in the production of a medicament for the treatment of depression.

2.   The use of (-)-2-amino-6-n-propyl-amino-4,5,6,7-tetrahydrobenzothiazol and its pharmacologically-acceptable acid addition salts in the production of a medicament for the treatment of depression.

**Revendications**

1.  Utilisation du 2-amino-6-n-propyl-amino-4,5,6,7-tétrahydrobenzothiazole ainsi que de ses sels d'addition d' acide pharmacologiquement acceptables pour la préparation d'un médicament pour le traitement des dépressions.

2.  Utilisation du (-)-2-amino-6-n-propyl-amino-4,5,6,7-tétrahydrobenzothiazole ainsi que de ses sels d'addition d'acide pharmacologiquement acceptables pour la préparation d'un médicament pour le traitement des dépressions.